# EUROPEAN PATENT APPLICATION

(11) **EP 4 174 866 A1**
(43) Date of publication of application: **03.05.2023**
(21) Application number: 21217403.1
(22) Date of filing: 23.12.2021
(51) Int. Cl.: G16H 10/40, G16H 10/60, G16H 15/00

(54) **USER-GUIDED STRUCTURED DOCUMENT MODELING**

(30) Priority: 27.10.2021 US 202163272355 P
(71) Applicant: Koninklijke Philips N.V., 5656 AG Eindhoven (NL)
(72) Inventor: MANKOVICH, Alexander Ryan, Eindhoven (NL); MALIK, Asad, Eindhoven (NL)
(74) Representative: Philips Intellectual Property & Standards

(57) **Abstract**

The present disclosure describes systems configured to guide users through a sequential mapping process to extract targeted information from received clinical report documents. The systems are configured to utilize the extracted information to generate a comprehensive, flexible report data model used to process incoming clinical report documents having the same document structure. Systems are uniquely configured to map incoming reports by utilizing the source code of PDF files, including the displayed text, information that determines how the text appears, and the absolute position of the text within each document constituting a clinical report.

## Description

### TECHNICAL FIELD OF THE INVENTION

The present disclosure pertains to systems and methods for guiding the capture of patient data from clinical reports and mapping the captured data to data models used to streamline the capture and integration of similar data from subsequent clinical reports.

### BACKGROUND OF THE INVENTION

The integration of clinical reports from various sources into more comprehensive medical systems continues to present many challenges despite significant advances in the generation and transmission of electronic medical records. Patient-specific genomics data, for example, may be included in a wide variety of clinical report structures, formats, and visual representations. PDF reports are common but highly customized from customer to customer, and even a single customer may have multiple internal versions of reporting structure. This variation limits the ability of report integration systems to efficiently receive and process clinical reports in a consistent, user-friendly manner.

Named Entity Recognition (NER), an application of Natural Language Processing (NLP), provides one solution for capturing clinical report content in a streamlined manner without expert-guided curation, but this approach requires vast datasets for adequate training and is ill-suited for extracting and categorizing specific information from unstructured text in highly customized document structures, especially when exact specificity is needed. The challenges posed to NER tasks usually differ between reporting types as well. Radiology reports, for example, have a relatively standard structure, but with diverse ways of expressing findings. Genomics reports, conversely, have entirely customized, laboratory-specific structures, but with relatively standard ways of expressing findings. Additional data capture mechanisms involving the application of technical standards to integrate assorted clinical reports are similarly limited and sparsely adopted.

Improved technologies are therefore needed to ingest incoming clinical reports from a variety of sources and integrate the resulting data into comprehensive, standardized models in accordance with user instructions.

### SUMMARY OF THE INVENTION

The present disclosure describes methods and systems configured to guide users through a sequential mapping process for a variety of clinical reports. Implementations involve generating and implementing a clinical report template and data capture mechanism applicable to a wide variety of clinical report types regardless of clinical domain. Flexible, comprehensive data models can be generated via guided mapping of electronic clinical reports, which can then be utilized to efficiently map information fields from subsequently received reports having the same structure.

In accordance with embodiments of the present disclosure, a method may involve displaying a first clinical report (308) having a type, wherein the first clinical report is in an unstructured electronic format. The method may also involve displaying, via a graphical user interface (GUI), a graphical user interface component, which is also referred to herein as a skeleton report template, (302a) that enables a user to select, from a plurality of elements (304a) of a reference information model (also referred to as default or pre-stored data model), a subset of the plurality of elements for inclusion in a custom data model for clinical reports of the type, wherein the GUI is further configured to enable the user to map unstructured information from the first clinical report (308) to the elements of the custom data model whereby the unstructured information is extracted from the first clinical report and stored in structured format, in a first converted clinical report compliant with the reference information model. The method may also involve parsing a second clinical report of the type using the previously-defined custom data model to generate a second converted clinical report compliant with the reference information model that contains, in structured format, the information from the second clinical report which was previously contained in the second clinical report as unstructured information. Upon creating a custom data model for clinical reports of the type, any subsequent ingestion of unstructured information in clinical reports of the type can be made more efficient or streamlined.

In some embodiments, parsing of the second clinical report of the type includes selecting the custom data model and the second clinical report, and information from the first converted clinical report, in connection with the custom data model, to automatically extract the non-machine readable information from the second clinical report and store the extracted information in machine-readable form in a second converted clinical report compliant with the reference information model. In some embodiments, parsing of the second clinical report of the type includes displaying the second clinical report, and, upon selection of the custom data model, enabling the GUI for mapping, responsive to user inputs, non-machine readable information from the second clinical report to the elements of the custom data model for generating the second converted clinical report.

In accordance with embodiments of the present disclosure, a computing system may include at least one processor and at least one memory storing instructions which when executed by the processor cause the computing system to display a graphical user interface configured to enable a user to select clinical information fields stored in a default data model. The computing system may also be caused to display a first clinical report document of a given type via a graphical user interface, the first clinical report containing corresponding clinical information fields. The computing system may be further caused to store computer-readable instructions for implementing a data ingestion tool and a data model generator via the processor. The data model generator may be configured to generate a clinical report data model by guiding a user through a sequential report mapping process. The data ingestion tool may be configured to utilize the clinical report data model to guide the user through a streamlined mapping process upon receipt of additional clinical reports.

In some embodiments of the computing system, the sequential report mapping process involves prompting the user, via the graphical user interface, to select the clinical information fields stored in the default data model and map the clinical information fields to the corresponding clinical information fields embodied in the first clinical report. In some embodiments of the computing system, the clinical information fields comprise patient information, clinical test results, diagnoses, symptoms, genetic mutations, treatments, and/or patient outcomes. In some embodiments of the computing system, mapping the clinical information fields to the corresponding clinical information fields involves determining coordinates of the corresponding clinical information fields within the first clinical report. In some embodiments of the computing system, mapping the clinical information fields to the corresponding clinical information fields involves determining relative positions between the corresponding clinical information fields within the first clinical report. In some embodiments of the computing system, the data model generator is further configured to prompt the user to assign an information field as an anchor point from which each of the remaining clinical information fields is mapped. In some embodiments of the computing system, mapping the clinical information fields to the corresponding clinical information fields involves determining font attributes of the corresponding clinical information fields within the first clinical report. In some embodiments of the computing system, the clinical report data model comprises a computer-readable model compatible with all clinical reports having the same document structure as the clinical report documents. In some embodiments of the computing system, the sequential report mapping process involves prompting the user, via the graphical user interface, to indicate whether the clinical information fields are required or optional. In some embodiments of the computing system, the first clinical report comprises genomics reports and at least one of the corresponding clinical information fields comprises a genetic mutation.

In accordance with embodiments of the present disclosure, a method of modeling and processing clinical report data involves transmitting clinical report document data to a computing device, receiving and displaying clinical report documents on a graphical user interface of the computing device, generating a clinical report data model by guiding a user through a sequential report mapping process, and utilizing the clinical report data model to guide the user through a streamlined mapping process upon receipt of additional clinical reports.

In some embodiments, the method further involves prompting the user, via the graphical user interface, to select clinical information fields stored in a default data model and map the clinical information fields to corresponding clinical information fields embodied in the clinical report documents. In some embodiments, the clinical information fields include patient information, clinical test results, diagnoses, symptoms, genetic mutations, treatments, and/or patient outcomes. In some embodiments, mapping the clinical information fields to corresponding clinical information fields involves determining coordinates of the corresponding clinical information fields within the clinical report documents. In some embodiments, mapping the clinical information fields to the corresponding clinical information fields involves determining relative positions between the corresponding information fields within the clinical report documents. In some embodiments, the method further involves prompting the user to assign an information field as an anchor point from which each of the remaining information fields is mapped. In some embodiments of the method, mapping the clinical information fields to the corresponding clinical information fields involves determining font attributes of the corresponding clinical information fields within the clinical report documents. In some embodiments, the clinical report data model comprises a computer-readable model compatible with all clinical reports having the same document structure as the clinical report documents. In some embodiments, the sequential report mapping process involves prompting the user, via the graphical user interface, to indicate whether clinical information fields are required or optional.

In accordance with principles of the present disclosure, custom (or report-specific) data models, generated through the sequential mapping process described herein, can advantageously be used to streamline (e.g., making it more efficient and less ad-hoc) the ingestion of data a large number and a variety of different clinical reports from various workflows or users, thereby effectively standardizing (e.g., in terms of format) these various clinical reports, and making the data contained therein available within a single medical information computing system (e.g., a medical information SaaS platform), which can reduce computational and human resources.

Any of the methods described herein, or steps thereof, may be embodied in a non-transitory computer-readable medium comprising executable instructions, which when executed may cause one or more hardware processors to perform the method or steps embodied herein.

### BRIEF DESCRIPTION OF THE DRAWINGS

Fig. 1 is a block diagram illustrating an example of a system for data model generation guidance implemented in accordance with embodiments of the present disclosure.
Fig. 2 is a flowchart of a data model mapping workflow implemented using the system depicted in Fig. 1 in accordance with embodiments of the present disclosure.
Fig. 3A is a schematic of a user interface configured to guide a user through a document modeling generation process in accordance with embodiments of the present disclosure.
Fig. 3B is another schematic of the user interface of Fig. 3A.
Fig. 4 is an example of data modeling programming code embodying a data modeling architecture for user-guided static element selection and mapping implemented in accordance with embodiments of the present disclosure.
Fig. 5 is an example of data modeling programming code embodying a data modeling architecture for user-guided nested static element selection and mapping implemented in accordance with embodiments of the present disclosure.
Fig. 6 is a snapshot of example metadata collected and mapped to describe the locational relationship between various objects in accordance with embodiments of the present disclosure.
Fig. 7 is a conceptual schematic illustrating the manner in which variable elements may be constructed and mapped to an anchor point in accordance with embodiments of the present disclosure.
Fig. 8A is an example of programming code corresponding to variable element object selection and mapping implemented in accordance with embodiments of the present disclosure.
Fig. 8B is a continuation of Fig. 8A.
Fig. 9 is a block diagram outlining the base classes that may be involved in the data model generation processes implemented in accordance with embodiments of the present disclosure.
Fig. 10 is a flowchart of a static element mapping workflow implemented in accordance with embodiments of the present disclosure.
Fig. 11 is a flowchart of a variable element mapping workflow implemented in accordance with embodiments of the present disclosure.
Fig. 12 is a simplified block diagram illustrating an example processor implemented in accordance with embodiments of the present disclosure.

### DETAILED DESCRIPTION OF EMBODIMENTS

Various embodiments are described more fully below with reference to the accompanying drawings, which form a part hereof, and which show specific exemplary embodiments. However, embodiments may be implemented in many different forms and should not be construed as limited to the embodiments set forth herein; rather, these embodiments are provided so that this disclosure will be thorough and complete, and will fully convey the scope of the embodiments to those skilled in the art. Embodiments may be practiced as methods, systems, computer programs, machine-readable mediums or devices. Accordingly, embodiments may take the form of a hardware implementation, an entirely software implementation, or an implementation combining software and hardware aspects. The following detailed description is, therefore, not to be taken in a limiting sense.

Reference in the specification to "one embodiment" or to "an embodiment" means that a particular feature, structure, or characteristic described in connection with the embodiments is included in at least one embodiment of the invention. The appearance of the phrase "in one embodiment" in various places in the specification are not necessarily all referring to the same embodiment.

Some portions of the description that follow are presented in terms of symbolic representations of operations on non-transient signals stored within a computer memory. Some portions of the description are directed to e.g. a computer program. These descriptions and representations are the means used by those skilled in the data processing arts to most effectively convey the substance of their work to others skilled in the art. Such operations typically require physical manipulations of physical quantities. These quantities may take the form of electrical, magnetic, or optical signals capable of being stored, transferred, combined, compared and otherwise manipulated. It is convenient at times, primarily for reasons of common usage, to refer to these signals as bits, values, elements, symbols, characters, terms, numbers or the like. Furthermore, it is also convenient at times, to refer to certain arrangements of steps requiring physical manipulations of physical quantities as modules or code devices, without loss of generality.

However, all of these and similar terms are to be associated with the appropriate physical quantities and are merely convenient labels applied to these quantities. Unless specifically stated otherwise as apparent from the following discussion, it is appreciated that throughout the description, discussions utilizing terms such as "processing," "computing," "calculating," "determining," "displaying" or the like, refer to the action and processes of a computer system, or similar electronic computing device, that manipulates and transforms data represented as physical electronic quantities within the computer system memories or registers or other such information storage, transmission, or display devices.

Certain aspects of the present invention include process steps and instructions that could be embodied in software, firmware, or hardware, and when embodied in software, could be downloaded to reside on and be operated from different platforms used by a variety of operating systems. Embodiments can comprise one or more applications available over the Internet, e.g., software as a service (SaaS), accessible using a variety of computer devices, e.g., smartphones, tablets, desktop computers, etc. The data ingestion tool described below, for example, can be delivered/distributed using a SaaS product.

The present invention also relates to at least one apparatus configured to perform one or more of the operations disclosed herein. This apparatus may be specially constructed for the required purposes, or it may comprise a general purpose computer selectively activated or reconfigured by a computer program stored in the computer. Such a computer program may be stored in a computer readable storage medium, non-limiting examples of which may include read-only memories (ROMs), random access memories (RAMs), EPROMs, EEPROMs, magnetic or optical cards, application specific integrated circuits (ASICs), optical disks, CD-ROMs, floppy disks, magnetic-optical disks, or any type of media suitable for storing electronic instructions, and each coupled to a computer bus. Furthermore, the computers referred to herein may include a single processor or may be architectures employing multiple processor designs for increased computing capability.

### Definitions

As used herein, "users" may include various medical professionals, clinicians, and personnel, non-limiting examples of which can include oncologists, radiologists, neurologists, cardiologists, etc. "Users" may also include system implementation engineers tasked with integrating received patient data with current data processing and/or viewing systems utilized by medical professionals. "Users" can also include researchers and/or archivists studying and/or storing patient- and/or population-specific medical data.

As used herein, "vendors" may include third-party suppliers of patient test results. In some examples, a vendor may include a genomic sequencing servicer equipped to obtain, annotate, store, and/or report raw sequences of genomic data. A genomic sequencing servicer, for instance, can also identify and report patient-specific mutations after aligning raw sequence reads to a reference sequence. Upon receipt of the sequencing data, e.g., genotypes, a user can determine its clinical relevance, for example based on one or more associated phenotypes and/or symptoms, and based on the determination, choose a treatment approach, which may be further informed by previously implemented workflows implemented for patients having similar genomic data.

While genomics reports are described herein, the "clinical reports" referenced throughout this disclosure may include a variety of report types in other clinical domains. The term clinical report may refer to any type of report, in electronic format (e.g., PDF format or another suitable file format) that contains medical information. The disclosed report template and data capture mechanisms are sufficiently generic to enable broad application across various report types. Accordingly, it should be understood that genomics reports are referenced herein for illustration purposes only and should not be viewed as limiting.

The term unstructured electronic format, as used to describe clinical reports of the present disclosure generally implies that some or all of the medical information contained in the report is not structured, and thus it cannot be imported or read by a computer, and may thus also be referred to as non-machine-readable. This is contrasted with structured electronic formats, such as comma-separated values (CSV), JavaScript Object Notation (JSON) or Extensible Markup Language (XML) formats, that data in which is necessarily structured, and thus it can be processed or "read" by a computer. These and other such structure formats or data can be referred to as machine-readable.

As used herein, the terms "unified model," "customized clinical report model," "final model," and "complete model" may be used interchangeably.

The described systems and methods support user-guided extraction and storage of select patient information by healthcare providers, administrators, and researchers to permit effective analysis of healthcare information at the patient and population level. In some examples, systems and methods disclosed herein can be integrated with various Enterprise Platforms within healthcare, hospitals and beyond. For instance, this could be Philips IntelliSpace platform. This allows to receive, interpret, and store clinical reports for ongoing patient analysis and retrospective review of treatments and outcomes in an improved manner. The improved workflow achieved via implementation of the disclosed technology can more accurately synthesize clinical information derived from a plurality of sources, streamline treatment processes by revealing best treatment practices for patients having a variety of clinical test results, improve user access to clinical information, and reduce human error in the collection and interpretation of patient data. While embodiments may be implemented in patient healthcare data systems and methods, they are not limited to this context, and may also be implemented in other document management systems.

Embodiments described herein may relate to a computing system (e.g., a SaaS platform) programmed to process and display multiple types of medical information. An example of such a computing system may be configured to process and display information related to cancer diagnoses and treatment options. Diagnostic information can include imaging data, genomic data, pathology data, patient-specific medical history, etc., all of which may also inform treatment decisions in view of evolving research findings. Patient outcomes can then be paired with the diagnostic information and treatment approach(es) to assess treatment effectiveness and determine best practices. Different types of electronic clinical reports having different document structures are received by a computing system according to the examples herein, which is configured, in some embodiments, to integrate and display the information derived from the reports in accordance with user preferences. Systems described herein may be configured to accomplish these tasks on a large scale with reduced manual curation relative to pre-existing systems.

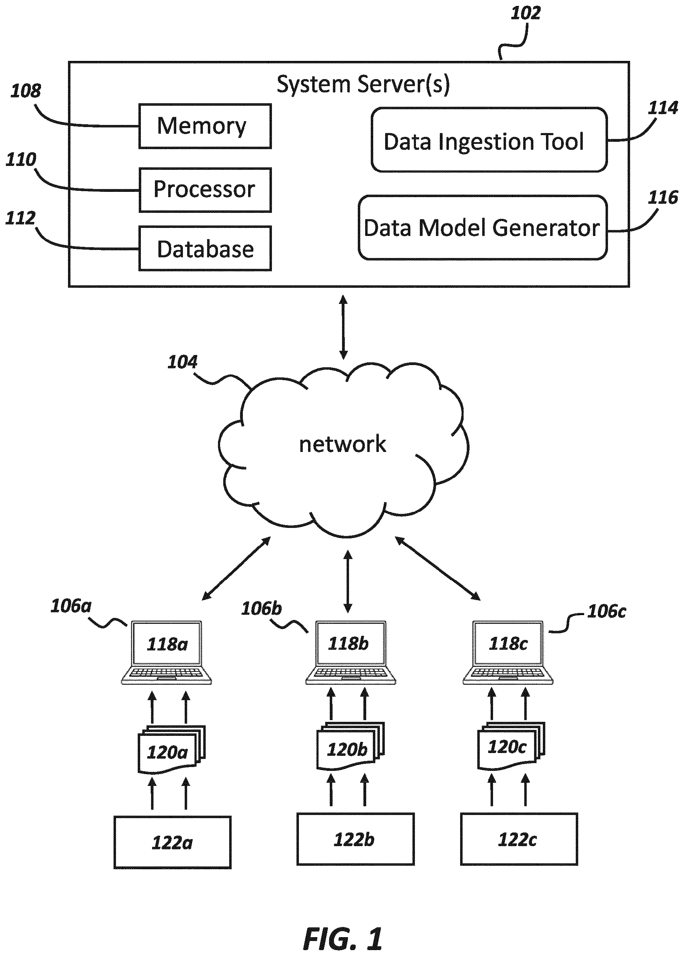

Fig. 1 is a block diagram illustrating an example of a system 100 for data model generation guidance implemented according to one or more embodiments disclosed herein. As shown, the system 100 can include one or more servers 102, which may be communicatively coupled via a network 104, and one or more user devices 106a,b,c (also referred to as client devices). The server(s) 102 include at least one non-volatile memory 108, and at least one processor 110. In some embodiments, the server(s) 102 may include or be in communication with a storage database 112, which may store previously received clinical reports, mapping templates, and/or data models. In some embodiments, one of the servers 102 may be configured as a storage server and may thus provide the storage database 112, which may be shared by one or more of the other servers 102, which may be configured as application servers, for executing processes associated with the data ingestion application (e.g., data model customization, etc.). In the example shown, the processor 110 of the example server 102 is configured to implement a data ingestion tool 114 and/or a data model generator 116, each of which may comprise a module embodying computer-executable instructions, data structures, routines, applications, or software programs stored in the memory 108 and configured to implement one or more actions described herein pursuant to a sequential mapping process used to guide a user through a document data modeling method. For example, the data model generator 116 may be configured to create and/or customize new data models based on user input and the structure of incoming clinical reports. The data ingestion tool 114 may be configured to guide users through a mapping process upon receipt of new clinical reports using the generated model(s), thereby bypassing one or more model customization steps. The system 100 may be implemented on or as one or more general purpose computers, special purpose computers, a programmed microprocessor or microcontroller and peripheral integrated circuit elements, an ASIC or other integrated circuit, a digital signal processor, a hardwired electronic or logic circuit such as a discrete element circuit, a programmable logic device such as a PLD, PLCA, FPGA, Graphics processing unit (GPU), or PAL, or the like. While described here as a distributed system including server-side and client-side devices, it will be understood that in some embodiments, the application(s) or portions thereof executed by the server processor 110 may instead be executed by a processor of a client device. In other words, in some embodiments, any of the functionality of applications described in the present example as server-side applications, may be hosted and executed by a client computing device and may not be executed in distributed manner, although even in such applications, one or more of the data models, clinical reports or other information consumed by the application(s) may be retrieved from one or more networked storage device(s). In some embodiments, one or more of the components shown in Fig. 1 can be separated or combined. The data ingestion tool 114 and data model generator 116, for example, can be combined and executed by the same processor according to some embodiments. In other embodiments, these sub-applications may be executed by different processors.

The one or more user devices 106a-c are communicatively coupled to the server(s) 102 via a network 104, and include one or more input/output devices (e.g., one or more displays, which may include a touch screen, a keyboard, mouse or other pointer device(s), or any combinations thereof) configured to present a graphical user interface 118a,b,c for receiving user input in connection with the execution of the data ingestion tool or application. In the example in which the server(s) 102 are in communication with multiple client devices, each client device may present, on its display, a respective graphical user interface 118a,b,c that enables the respective user to view options associated with the default data model and customization thereof, and to select various information fields, e.g., patient age or diagnosis, within a displayed clinical report in connection with the mapping process. The user devices 106a,b,c can retrieve a clinical report from its local memory, from a memory device (e.g., the database) of the server 102, or may receive clinical reports 120a,b,c from a variety of vendors 122a,b,c,d and/or internal workflows. An initial clinical report of a given type may be presented on the client device for customization of the data model associated with the given type of clinical report, following which subsequent clinical reports of the same type ingested by the system 100 may bypass the model customization steps described herein for more efficient extraction of data therefrom. The number of client devices and vendors can vary in different examples. Each of the client devices 106a,b,c may be implemented by any suitable computing device such as a tablet mobile device, a handheld mobile device, a smart phone, a wearable mobile device, a desktop or a laptop network device, etc., configured to communicate over the network 104.

The network 104 may be substantially any type of network (wired, wireless or combinations thereof) which utilize any suitable system or protocol (or combinations of systems and protocols) that provide for data exchange between the computing devices in the system 100, including both wired and wireless communication technologies. For example, the network 104 may include Wi-Fi, Bluetooth, cellular networks, Ethernet, or other suitable network systems, e.g., cloud networks.

The server(s) 102 can be implemented by any suitable type of computing device, in some embodiments including one or more computing devices in communication with one another that collectively perform one or more methods disclosed herein, also referred to herein as distributed computing. In some embodiments, the server 102 is a computing device that hosts a web server application or other software application that transmits and receives data to and from the client devices 106a,b,c. In some embodiments, certain aspects of the web server application hosted by server(s) 102 may be performed on the client device(s) such as collection of user inputs associated with the customization of the data models described herein. In addition to those shown in Fig. 1, the server 102 can include a variety of processing elements, memory components, and networking/communication interfaces, and may generally have increased processing power and memory storage relative to the viewer devices 106a,b,c. Each of the computers constituting the server 102 requires a network connection and power source to operate, and each may include redundant components for power and interfaces.

The server 102 is configured to host one or more aspects of the data modeling guidance system 100 disclosed herein, such as the data ingestion tool 114, which is configured to implement a sequential mapping process based on received user input and the targeted capture of various document attributes which, in tandem with the data model generator 116, creates a flexible report data model.

The memory 108 may be implemented by any suitable computer-readable medium on which data (e.g., program code and any associated data or data upon which the executed program acts or which is generated by the execution of the executed program) can be stored in a format that can be read by a machine, such as a disk, hard drive, or the like. Common forms of computer-readable media include, for example, floppy disks, flexible disks, hard disks, magnetic tape, or any other magnetic storage medium, CD-ROM, DVD, or any other optical medium, RAM, ROM, PROM, EPROM, FLASH-EPROM, variants thereof, other memory chip or cartridge, or any other tangible medium from which the processor 110 can read and execute. The memory 108 can include or be coupled with one or more data storages utilizes by a network device to store applications and data, which may include data models and configurations thereof.

In some embodiments, the clinical reports 120,b,c may include portable document format documents (PDFs) lacking underlying mark-up data, the absence of which typically impedes the identification and extraction of patterns from the documents that could otherwise be used to extract data from incoming reports in a consistent manner with little to no manual curation. In some embodiments, clinical reports 120a,b,c are often supplied by vendors 122a,b,c in machine-readable formats that lack the mark-up data, which limits the ability of pre-existing systems to extract the data embodied therein.

Generally, the components of the system 100 are configured to implement a sequential mapping process that leverages user input with natural language processing to extract targeted information from the clinical reports 120a,b,c. The system 100 is configured to standardize and unify various types of clinical reports 120a,b,c received by a wide variety of users, and unlike pre-existing systems, the system 100 may be readily scalable and robust in its support and integration of diverse clinical reports. The system 100 can also reduce user reliance on costly, ad hoc generation of manually defined report mapping templates.

The system is uniquely configured to utilize the source code of PDF files, which includes displayed text, information that determines how the text appears, and the absolute position of the text within each document. In some embodiments, the system disclosed herein can guide a user through a sequential mapping process that involves capturing this information to create custom mapping templates for previously acquired and newly incoming clinical reports. Additionally, the system is configured, in some embodiments, to receive user input that is further utilized to guide the user through the clinical report ingestion process, the end result being a customized clinical report model configured to receive and process a wide variety of clinical reports, differing in terms of both content and document structure.

Data models generated in accordance with the present disclosure can also be used to develop report-version-specific parsing mechanisms. For instance, when document mapping is complete and the unified data model has been generated, the additional coordinate/font attribute/relative mapping elements that were collected along with the user selections can be used to approximate where the desired elements are in a new report having the same version/structure as the report used to build the unified model. With the parsing mechanism in place, a single curation event can result in a parsing mechanism configured to automatically produce many data points, thereby reducing the number of curation events while still enabling the creation of a robust research database. This marks a significant practical improvement relative to pre-existing systems that require a separate curation process for each stored or incoming clinical report.

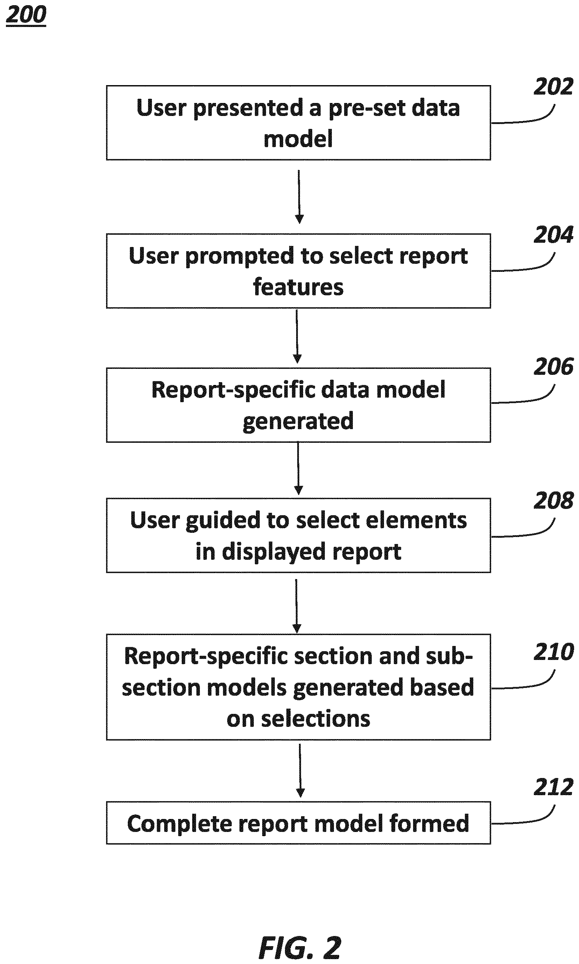

Fig. 2 is a flowchart of a mapping workflow implemented by the system 100 to construct a custom, report-specific data models based on user input and/or document attributes, e.g., document headers, absolute and relative text locations, text sizes, fonts, etc. At step 202, the system 100 may present a pre-set data model to a user on one of the graphical user interfaces 118a,b,c. At step 204, the system 100 may prompt the user to select certain report features, for example by presenting the user with populatable entry fields or selections based on the pre-set, default data model constructed using a pre-existing data model or class. In some embodiments, selectable features can include labels and corresponding data that will appear in a final clinical report. Non-limiting examples of such features may include report type, medical record number (MRN), patient name, patient diagnosis, single nucleotide polymorphisms, gene fusions, and/or other features present within a clinical report received from a vendor.

At step 206, the system 100 may generate a custom data model based on the selected report features. Alterations to the custom data model can be made throughout the mapping process. Such alterations can include adding or removing data, or making certain features optional or required.

At step 208, the system 100 guides the user to select the elements in a displayed clinical report. The elements may correspond to the aforementioned labels and associated data, thereby mapping the selected features from the default data model to the same features in a clinical report document. These selections may be used to populate report-specific models and subsection data models at step 210, both of which can be used to store the information related to the selected elements, including their position in an actual clinical document and any associated font attributes.

At step 212, the system 100 forms a complete report. The complete report could be optionally displayed. After the user has processed the entire report, the model generator 116 can generate a complete report model based on the collection of underlying models. The complete report model embodies the computer-readable model compatible with all clinical reports having the same structure as clinical report. In various embodiments, the data model may be defined and stored in various computer programming languages, non-limiting examples of which may include C, C++, Perl, Python, Java, JavaScript, JavaScript Object Notation (JSON), etc. The data model may include information categorized into labels, sections, data, coordinates, and/or font attributes. In various embodiments, the labels can comprise categorical features typically included in a clinical report, including data headers such as "Name" and "Age." The data can comprise values corresponding to the labels, e.g., "John Doe" and "43," respectively. The sections can comprise broader document-level headers, e.g., "Patient Information." The coordinates can comprise the position of the aforementioned features within the incoming clinical report document. The coordinates are used to map the location of the data relative to their corresponding labels or other reliable anchor points. In some examples, an approximate coordinate mapping may be implemented, which allows for a margin of positional error, for example +/- one or more pixels. Font attributes determine how the text appears, e.g., font type, font size, etc.

By implementing the mapping workflow depicted in Fig. 2, or an embodiment thereof, the system 100 may bypass or reduce user reliance on implementation engineers to manually define report mapping templates for subsequent PDF parsing in a manner compliant with pre-existing data models.

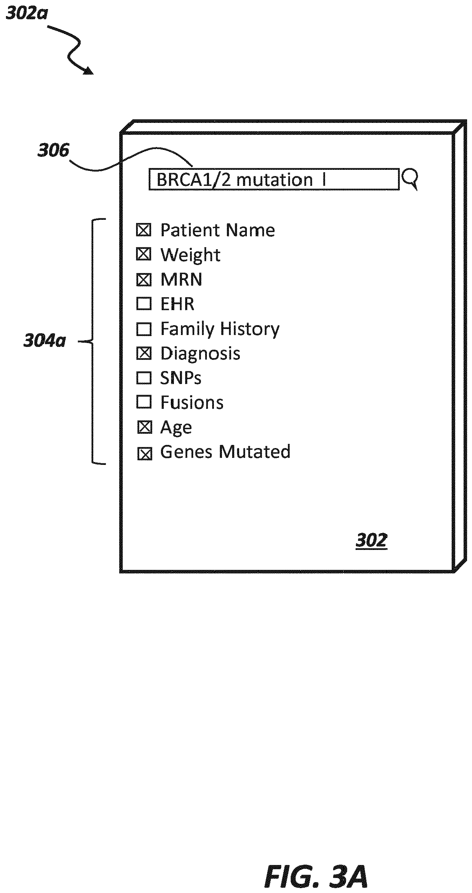

Fig. 3A illustrates a simplified example of a graphical user interface 302 displaying a graphical user interface component, which is also referred to herein as skeleton report template, 302a configured to enable the collection of user input for the generation of a custom (or interchangeably report-specific) data model in accordance with embodiments disclosed herein. The graphical user interface (GUI) component 302a may be presented on a display (e.g., of a client computing device) to enable the user to select a subset from the plurality of elements of the reference information model to be included in the report-specific data model, which may facilitate the downstream development of a parser for a given type of input clinical report that makes the extraction or ingestion of information from subsequent clinical reports of this type more efficient. Customization of the final data model may involve displaying a clinical report of a given type in conjunction with a graphical user interface which enables the user, in some cases guiding the user through prompts, to perform a stepwise mapping process that results in the creating of a document-specific data model containing user-selected features, document attributes, and positional feature interrelationships. As shown in Fig. 3A, the GUI component 302a can be configured to display selectable report features 304a, such as patient name, weight, MRN, etc., corresponding to elements of a default (or pre-stored) data model. The GUI component 302a may display a keyword entry field 306 which may be associated with a drop-down menu, such that a user can enter a text string in the field 306 and if the text string matches data entries associated with the drop-down menu, the corresponding matching data entry may then be displayed by a dropdown menu and added as a selected feature for the skeleton data model. The default data model may thus include a larger number of available report features than may be ultimately included in a skeleton data model for a given report type. When the desired report features for the skeleton data model have been selected, the user can proceed to subsequent steps of the customization process, e.g., by clicking, touching, or otherwise selecting a designated field in the graphical user interface, e.g., an "accept" or "continue" button of the GUI component 302a. The user input provided via the GUI component 302a is used by the system (e.g., processor 110) to generate a skeleton data model, which may include an appropriate number of sub-models or data structures for each selected report feature.

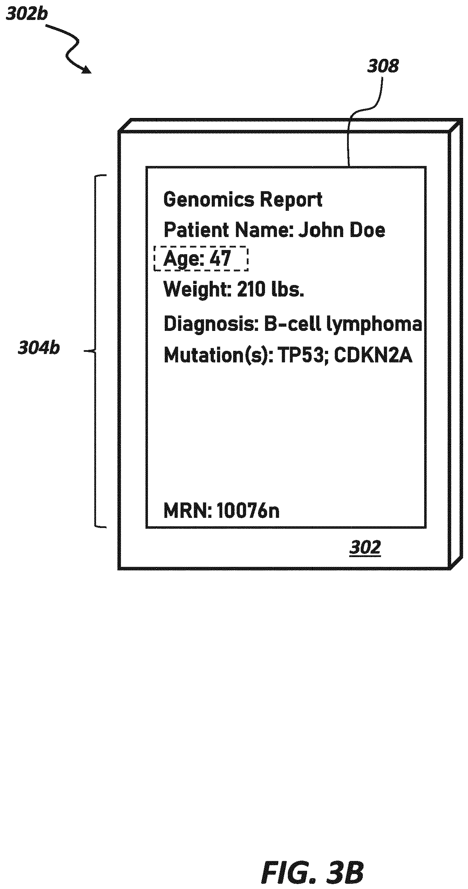

After or concurrently with the display and selection of the report features 304a from the default data model, the user can engage directly with the clinical report 308 by clicking, touching, or otherwise selecting the same features within the report. As shown, for example in Fig. 3B, the graphical user interface is further configured, via a GUI component 302b, to enable the user to select portions of a displayed electronic clinical report 308 to facilitate the mapping process. The GUI component 302b may include various tools or icons, such as a selection tool or icon, a text field or image/snapshot capture tool or icon, etc., to enable user inputs for mapping varies portions of the document (e.g., information fields) to corresponding elements of the data model. In the example shown in Fig. 3B, the user has selected patient age, before or after selecting the corresponding data model element such that the selecting information from the clinical report 308 can be mapped to the data model.

The selections may then be transmitted to a processor (e.g., processor 110 of server 102 or a processor of a client device which displays the GUI including components 302a and 302b) implementing the data model generator 116 to generate a custom data model based on the selections and their corresponding coordinates and attributes within the clinical report 308, for example by using the selections to determine coordinates and inserting the coordinate in an object definition. Subsequent clinical reports received by a client device can be transmitted to the server 102, after which the processor 110 can implement the data ingestion tool 114 to process the reports using the positional and attribute information stored in the custom data model.

In this manner, the user interface 302 can prompt users to efficiently and accurately retrieve targeted clinical information, which may uncover previously unrecognized clinical associations within a patient population and facilitate the identification of clinical manifestations that can inform patient sample selection for research and clinical trials, all of which can be achieved regardless of the specific orientation and layout of clinical information in the received documents. Embodiments may also enable effective navigation to sections and sub-sections within clinical reports containing information relevant to particular search interests. More generally, creating a custom data model as described herein creates in effect a custom workflow for making retrospective clinical reports machine readable (e.g., by converting unstructured data from the retrospective clinical reports into a structure data format that is compatible with the medical information computing system in which the medical data is ingested. Moreover, structuring the data in the retrospective clinical reports in this manner may further facilitate database curation, such as by placing the structured data (e.g., organized by attributed) into a database, which may be used (e.g., queried) for various purposes (e.g., clinical research).

The final data model may be composed of one or more data model objects, each of which may include a hierarchy of informational fields that correspond to the data each data model object represents. The data model can include labels, sections, data, coordinates, and/or font attributes of each user-selected feature that collectively facilitate classification of targeted information into two object classes. The first object class can comprise static elements defined by one-to-one relationships between labels and their corresponding data, and the second object class can comprise variable elements having a single label and an unknown, variable length of corresponding data points.

Static element mapping may require only the content, coordinates, and font attributes of each static element. For instance, a user may select a label element, which is then used to extract the coordinates of the selected label in the incoming report document, the text representing the label and the font attributes of the text. The row of the document containing the label can also be identified. The identified row and coordinates enable tracking of the positions of other elements surrounding the label in the document. The user can further identify the data element that corresponds to the identified label element. This identification is also used by the data ingestion tool 114 to extract the corresponding coordinates of the selected data in the report document, the text representing the data, the font attributes of the data, and the row of the document containing the data.

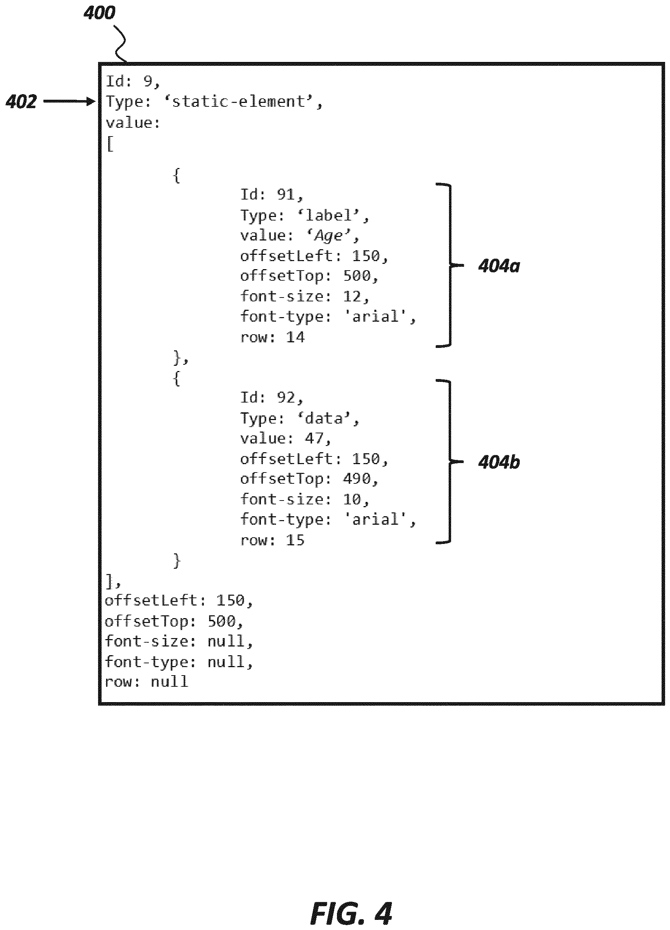

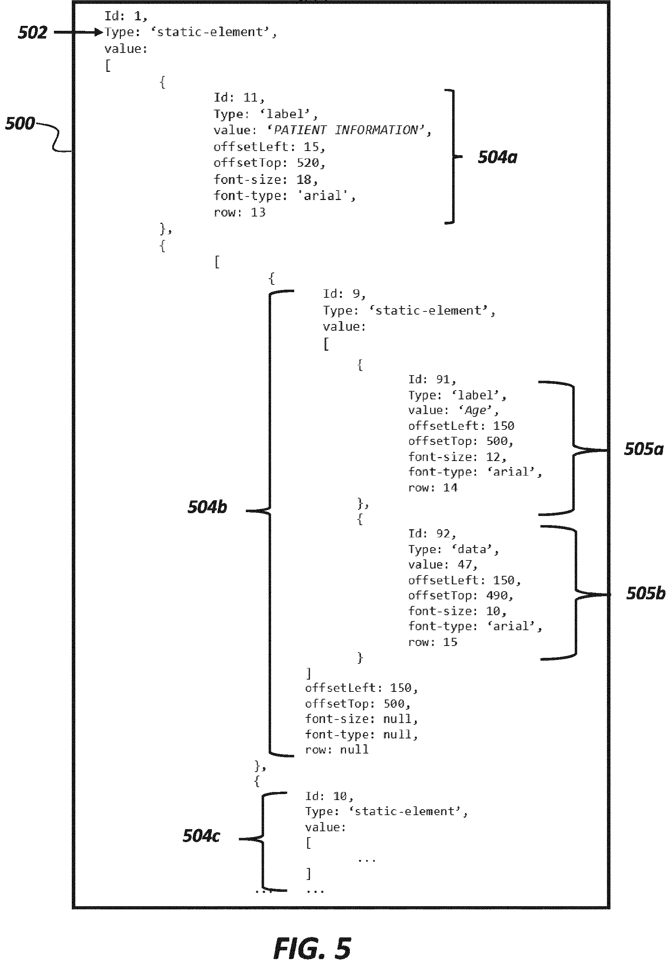

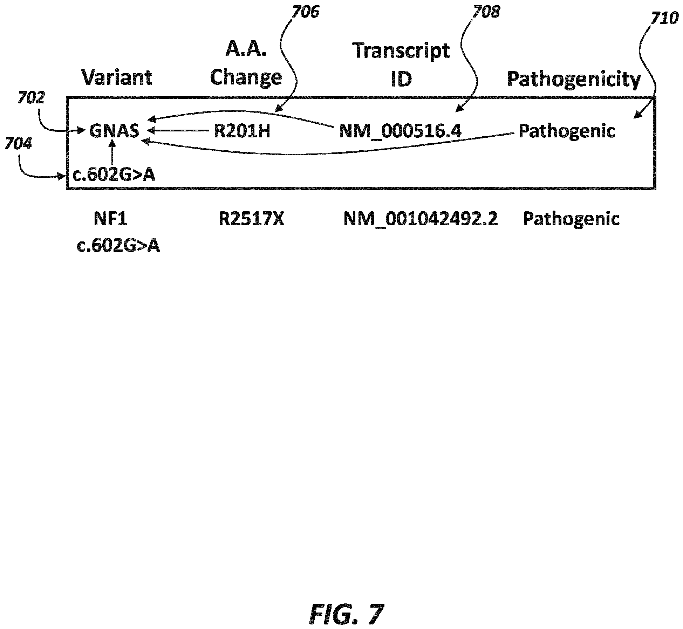

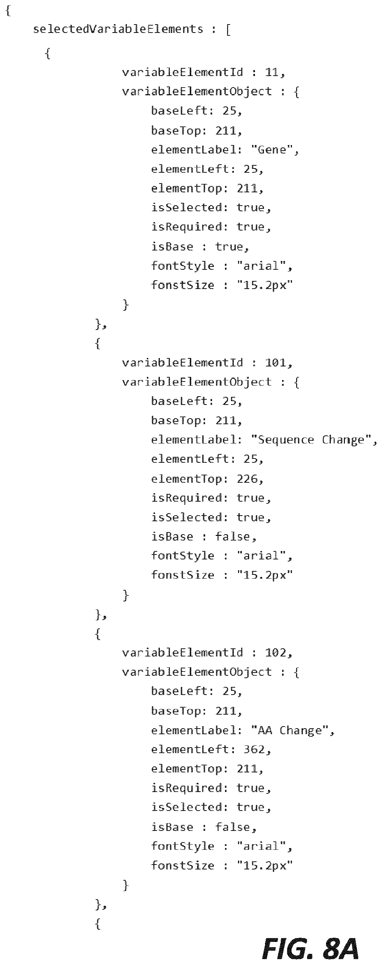

An example of a portion of a data model program code is shown in Fig. 4. The data model may be implemented using any suitable programming language, e.g., any of a variety of known scripting languages such as PHP, Python, C#, C+, C++, Java or other suitable programming language, and may be stored as language-specific program code (e.g., a script of the respective language used for the implementation of the data model) or in a language independent data format such as the JavaScript Object Notation (JSON) data format or another data format. As noted above, a data model according to the present disclosure may include any number of data model objects (or simply objects) corresponding to the desired number of data items to be extracted from a given clinical report type. In the example in Fig. 4, a simplified example of a single static object 400 is shown. The object 400 in this example includes multiple attributes, such as ID, type, value, offsets, and font-defining attributes. Multiple objects may be nested to define the properties of a given object, such as the static object 400 shown in Fig. 4, and/or nested together to define an object class of related objects, e.g., as shown in the example in Fig. 5. Each data model object may be associated with various properties, the values of which are customized as part of the mapping process. The data model object 400 in Fig. 4 is shown, for illustration purposes only, as having two properties 404a and 404b, each of which defines the location of two data fields within the clinical report. The example data model object 400 in Fig. 4 is a static object. A data model according to the present disclosure may include any number, in any suitable combination, of static and dynamic data model objects, an example of the latter being described further below with reference to Figs. 7 and 8A-8B. Each data model object may be associated with a set of attributes (e.g., Id, type, value, one or more offset attributes, and one or more font attributes), some of which may be required and some of which may be optional. In the example in Fig. 4, the data model object 400 has an Id attribute of 1, and type attribute 402 of object 400 is "static-element." The data model object 400 may thus be referred to as a static type object. Some of the attributes may be optional and may be assigned a null value if not specified. Some of the attributes may be specified by a user via the graphical user interface 302 (e.g., via the mapping processes), while some attributes are automatically assigned/defined by the system (e.g., through the creation of the template model). The value attribute of a data model object may be defined via one or more nested objects. In this example, the value of the static object 400 is defined by the two nested objects, namely first object 404a and second object 404b. The first and second objects are used to store related data (e.g., a label and data associated with the label, and respective coordinates within the clinical report) within the larger static object 400. Similar to object 400, each of the nested (or related) objects 404a and 404b has multiple attributes, some of which may be required (e.g., the Id, type, value, and one or more coordinate attributes) while some may be optional. In the illustrated example, the object 404a is of the type "label" and its value is defined as "Age" responsive to user input, e.g., either by direct user input (e.g., text entry) or via the mapping process. Similarly, other attributes such as the offset and font-related attributes may be defined responsive to user input (e.g., via the mapping process). The object 404b in this example is of the type "data" and its value is "47." Coordinates attributes may be defined in any suitable manner, for example by including a first coordinate attribute that defines the offset (e.g., from the left) to that particular data field, shown here as the attribute "offsetLeft" and a second coordinate attribute that defines the offset (e.g., from the top) to that particular data field, which is shown in this example as the attribute offsetTop. The objects 400, and its nested object(s) may include one or more font attributes and optionally a row attribute which may specify in which row of the document (e.g., the clinical report X) the particular data field appears. While only two objects 404a and 404b are shown here as defining the value attribute of the static object 400, in other examples fewer or greater number of related objects may be grouped into an attribute of a static object depending on the information to be extracted from a clinical report of a given type.

In some embodiments, multiple static objects can be nested to support complex data structures within received clinical reports, e.g., to enable the capture of section- and subsection-level information from a clinical report. A portion of a data model's programming code embodying such a nested architecture is shown in Fig. 5. Here, the value attribute of the data model object 500, which is of the type 502 "static-element," is defined by one or more nested objects, in this example including first object 504a with an Id attribute of 11, a second object 504b with an Id attribute of 9, a third object 504c with an Id attribute of 10, and so on. The second and third objects 504b and 504c, respectively, are used to represent data fields related to a same section of the report, represented here by the first object 504a. The second object 504b is also defined as a "static-element" object, whose value is defined by two sub-objects 505a and 505b. Similarly, the third object 504c is a static object and may include one or more sub objects for defining its value attribute. Multiple nested layers may be used in a data model according to the present disclosure, in which one or more static and or dynamic elements are nested, to reflect any desired complex structures (e.g., one or more sections, subsections, sub-subsections, and so on) of the received clinical report(s).

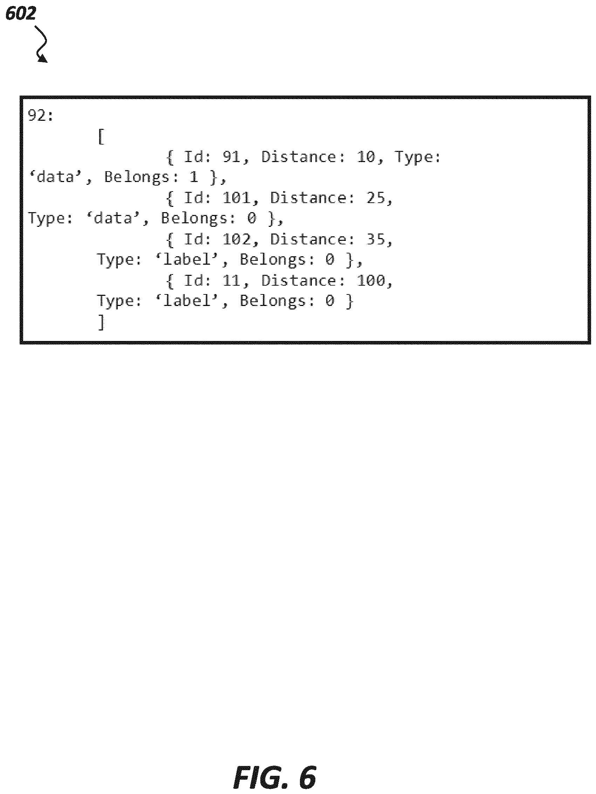

In some embodiments, as shown in an example in Fig. 6, metadata 602 may be generated, which describes the locational relationship between various objects of the data model. The metadata object 602 for the object with Id 92 is presented (this referenced object can be observed in Fig. 4 and Fig. 5 as objects 404b and 505b respectively). The metadata object describes objects proximal to the target object (in this case object with Id 92) and whether the target object is related to its proximal objects via the "Belongs" attribute. There are four proximal objects in metadata object 602, and each is described by their Id, Distance (e.g. Euclidean or otherwise computed metric for precise or approximate distance from the object of interest), Type (the data type of the object), and Belongs (i.e., whether the object of interest is meaningfully linked to the proximal object). The Belongs attribute value of 1 for proximal object 91 in metadata object 602 indicates that object 92 belongs to proximal object 91 (i.e., object 92 with attributes of {Type: 'data' and Value: 47} belongs to the object 91 with {Type: 'label' and Value: 'Age'}). A value of 0 indicates no relationship (e.g. object Id 101 with {Type: 'data'}, perhaps representing the data value belonging to the 'MRN' label), whereas a value of -1 indicates the opposite relationship (e.g. the Belongs value of object Id 92 within the metadata object for object Id 91, the inverse relationship in the figure). This attribute can be used to explicitly determine the relationship between objects and infer overall document structure. The metadata object can be generated for each object selected in the document and may span either locally (within a threshold distance from the object to capture those closest objects) or globally (to capture each object's relationship to all other objects).

In some embodiments, a data model may include one or more objects that include variable elements. Variable elements may be defined in relation to an anchor point and may include additional layers of user input(s) and/or document mapping. These additional layers are included to address challenges associated with processing undefined numbers of elements. Modifications may be necessary, for example, to enable a subsequent parsing mechanism to have the flexibility necessary to consistently capture information in a clinical report that includes a section containing three elements and another clinical report that includes ten elements for the same section. In embodiments of the variable element workflow, a user may first identify elements included in a clinical report and designate the elements as being required or optional. The relative relationship between variable data attributes and the location of a gene symbol, for example, may be critical to determine and identify a block of data to be extracted.

Fig. 7 shows the manner in which elements may be constructed and mapped to an anchor point in some examples. As shown, the anchor point 702 can consist of a gene symbol, e.g., GNAS. Via a graphical user interface, the user may label one or more related data points, which in the illustrated example include a specific nucleotide change 704 in the GNAS gene, the associated amino acid change 706, the associated transcript ID 708 of the genetic sequence containing the mutation, and the pathogenicity 710 of the mutation. The position of each user-selected data point is then mapped relative to the position of the anchor point 702, as indicated by the arrows, such that the coordinates of each element are identified and recorded. The same process may be implemented by the systems disclosed herein for the NF1 gene, as shown in the row below GNAS. The metadata utilized for variable element mapping can be used for multiple downstream applications, such as document parsing, pursuant to which a user can scan through a new clinical report of the same type and search for data points organized in a similar manner.

After indicating the targeted elements for inclusion in a final report, the user can select those elements in the received clinical report, further indicating whether each element is required or optional. The user then assigns one of the selected elements as the anchor point.

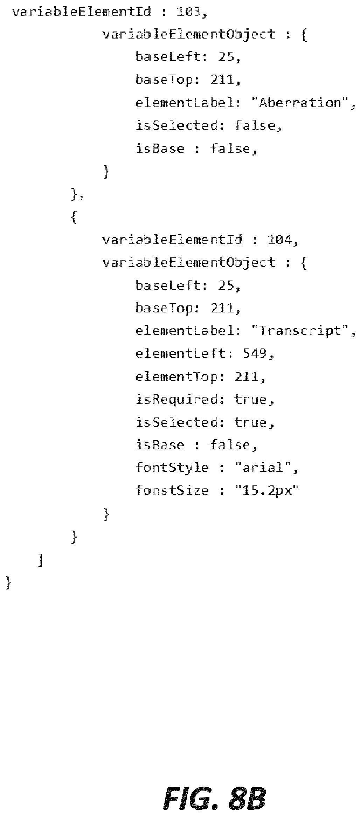

An example of programming code corresponding to variable element object selection and mapping is shown in Figs. 8A and 8B (Fig. 8B being a continuation of 8A). As shown, the coordinates of the base element are identified, followed by the type of label that has been selected by the user, the coordinates of which are also identified and indicated as "baseLeft, baseTop." Whether each element can be expected to be found in every instance of a data object is also identified and indicated as "isRequired." Whether an element is the base data point from which every other required data is mapped to is identified and indicated as "isBase." Font attributes for each element are also identified.

In the illustrated example, the first variable element is identified as variableElementId 11, which consists of a gene that is required and serves as the base point from which other variable elements are mapped. This particular variable element appears as text in Arial, 15.2 point font. As further shown, the second variable element variableElementId 101 consists of a sequence change that is required and selected, but is not the base element. It also appears as text in 15.2 point Arial font. The third object is identified as variableElementId 102. This object comprises an amino acid change that is required but is not the base element. The fourth object comprises an aberration, which is not selected and is not the base element. The fifth object comprises a required, selected sequence transcript.

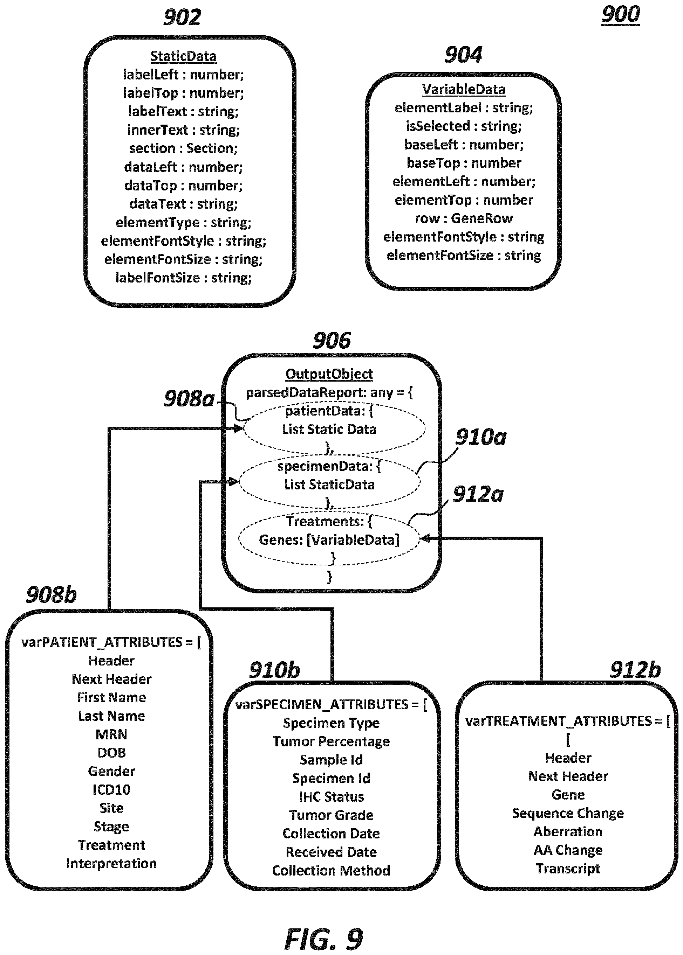

Fig. 9 is a block diagram outlining the base classes that may be involved in the information mapping processes described herein. Two classes are shown in the example in Fig. 9, including a static data class 902 for representing data points or elements having one-to-one relationship with their corresponding labels, and a variable data class 904 for representing data points or elements with multiple-to-one relationships with corresponding label(s). The OutputObject 906 in Fig. 9 is an example of an intermediate output model, which is an object, the top-level elements of which have been taken from the reference information model. The OutputObject 906 in the illustrated example includes patient data 908a, specimen data 910a, and treatments (or findings) 912a. In this example, patient data 908a and specimen data 910a are a collection of static data elements, whereas treatments 912a is a collection of variable data elements. Example listings of attributes or input elements that can be included in the patient data 908a, specimen data 910a, and treatments 912a are shown in blocks 908b, 910b, and 912b, respectively. Not all of the attributes/elements may be used in all embodiments, and additional attributes/elements may be added in yet other embodiments, with every additional element having the same functionality for labeling and parsing as the previously defined elements. The collection of static and/or variable data may be represented by objects defined by the workflows described previously, e.g., with reference to Figs. 4-8B.

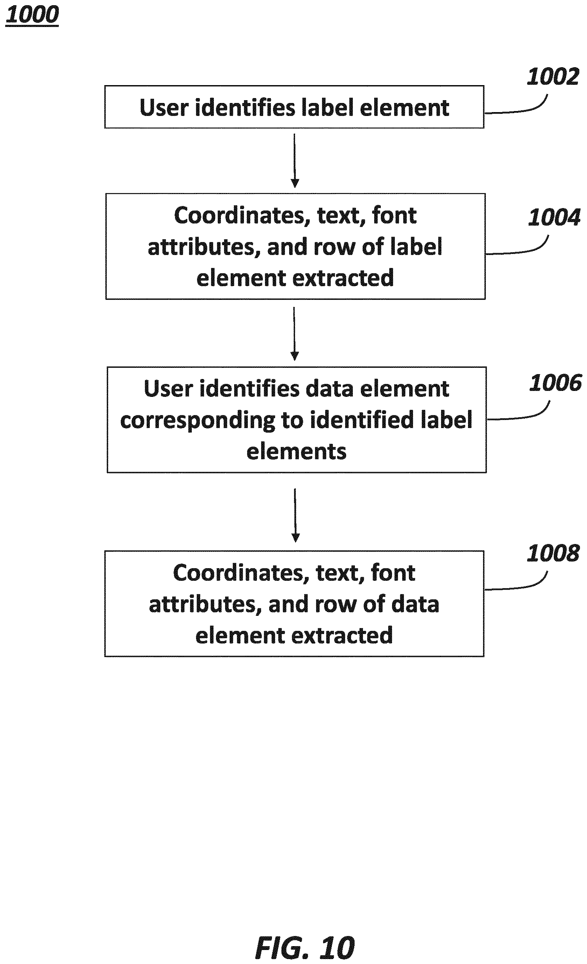

Fig. 10 is a flowchart of a static element workflow 1000 implemented in accordance with embodiments disclosed herein. At step 1002, a disclosed system prompts a user to identify a label element. At step 1004, a disclosed system extracts the coordinates, text, font attributes, and row of the label element within a clinical report. At step 1006, a disclosed system prompts the user to identify the data element corresponding to the label element. At step 1008, a disclosed system extracts the coordinates, text, font attributes, and row of the data element from the clinical report.

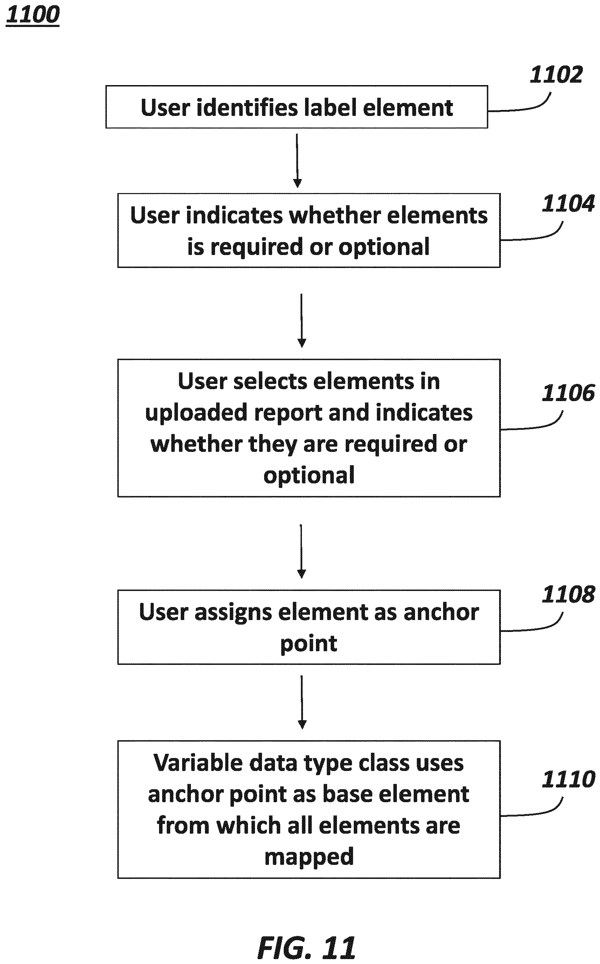

Fig. 11 is a flowchart of a variable element workflow 1100 implemented in accordance with embodiments disclosed herein. At step 1102, a disclosed system prompts a user to identify a label element. At step 1104, a disclosed system prompts the user to indicate whether elements are required or optional. At step 1106, a disclosed system prompts the user to selects corresponding elements in the uploaded clinical report and whether they are required or optional. At step 1108, a disclosed system prompts the user to assign an element as an anchor point. At step 1110, a variable data type class uses the anchor point as the base element from which all elements are mapped.

As a qualitative example of the manner in which the disclosed technology can be incorporated into a practical application of clinical record integration and display, a university or other research-oriented hospital or institute endeavoring to creating a document curation workflow may implement embodiments of the systems and methods described herein. Research hospitals often possess thousands of retrospective clinical genomics reports in PDF format that would be best utilized if integrated into a common database for subsequent analysis. Pre-existing technologies are configured to allow simple text annotation, along with the labeling of elements and sections with the stored documents, but are not configured to support the viewing and annotation of PDF documents or the collection of positional metadata therein. Unlike such systems, the disclosed technology can include a user interface configured to enable the research hospital to create a reference information model that serves as a unified model to which all retrospective clinical reports can be mapped. A user can upload a new clinical report to a disclosed system, which can then be displayed on a user interface. The user can then select, via the user interface, which elements of the unified model will appear in the final clinical report stored for current and/or future reference. This selection creates a custom data model comprised of empty values for the input clinical report. The user then begins selecting elements as they appear on the input report, and mapping them to the custom data model. Once all mappings are complete, the completed data model is saved in the system, including positional and PDF metadata to be used for downstream applications. The saved model can be compatible with all reports of the same version, such that when a user uploads another report of that same version, rather than selecting elements of the reference information model that appear, they can proceed straight to mapping the new report elements to the existing reference information model. The disclosed systems are thus configured to significantly reduce the need for time consuming, expensive document curation. Exemplary non-research hospitals also may benefit from this invention.

Additional NLP techniques can be applied to one or more of the aforementioned embodiments to further improve the generation of the unified model related to, for example, the detection of domain-specific attributes, e.g., gene symbol, thereby improving the overall quality of the resulting unified models.

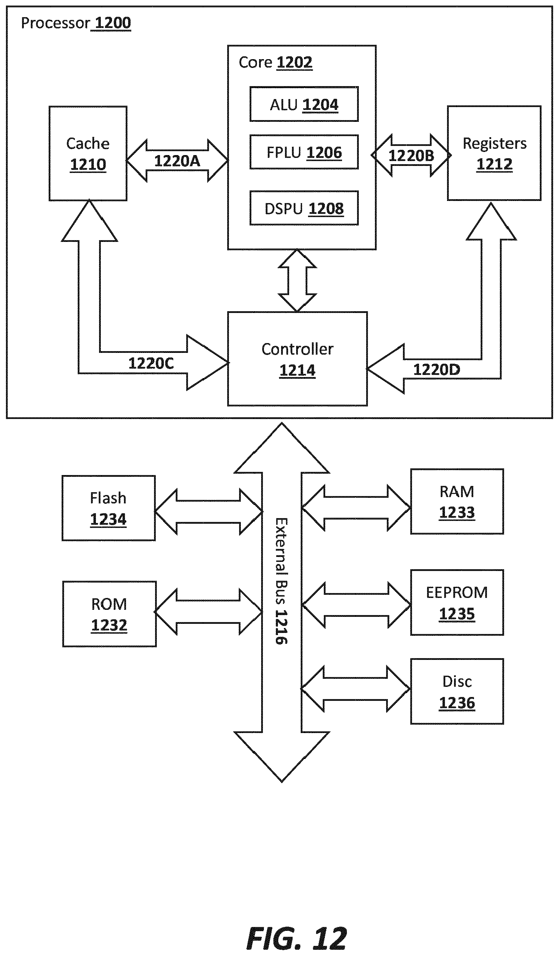

Fig. 12 is a simplified block diagram illustrating an example processor 1200 according to principles of the present disclosure. One or more processors utilized to implement the disclosed embodiments may be configured the same as or similarly to processor 1200. Processor 1200 may be used to implement one or more processes described herein.

Processor 1200 may be any suitable processor type including, but not limited to, a microprocessor, a microcontroller, a digital signal processor (DSP), a field programmable array (FPGA) where the FPGA has been programmed to form a processor, a graphical processing unit (GPU), an application specific circuit (ASIC) where the ASIC has been designed to form a processor, or a combination thereof.

The processor 1200 may include one or more cores 1202. The core 1202 may include one or more arithmetic logic units (ALU) 1204. In some examples, the core 1202 may include a floating point logic unit (FPLU) 1206 and/or a digital signal processing unit (DSPU) 1208 in addition to or instead of the ALU 1204.

The processor 1200 may include one or more registers 1212 communicatively coupled to the core 1202. The registers 1212 may be implemented using dedicated logic gate circuits (e.g., flip-flops) and/or any memory technology. In some examples the registers 1212 may be implemented using static memory. The register may provide data, instructions and addresses to the core 1202.

In some examples, processor 1200 may include one or more levels of cache memory 1210 communicatively coupled to the core 1202. The cache memory 1210 may provide computer-readable instructions to the core 1202 for execution. The cache memory 1210 may provide data for processing by the core 1202. In some examples, the computer-readable instructions may have been provided to the cache memory 1210 by a local memory, for example, local memory attached to the external bus 1216. The cache memory 1210 may be implemented with any suitable cache memory type, for example, metal-oxide semiconductor (MOS) memory such as static random access memory (SRAM), dynamic random access memory (DRAM), and/or any other suitable memory technology.

The processor 1200 may include a controller 1214, which may control input to one or more processors included herein, e.g., processor 110. Controller 1214 may control the data paths in the ALU 1204, FPLU 1206 and/or DSPU 1208. Controller 1214 may be implemented as one or more state machines, data paths and/or dedicated control logic. The gates of controller 1214 may be implemented as standalone gates, FPGA, ASIC or any other suitable technology.

The registers 1212 and the cache memory 1210 may communicate with controller 1214 and core 1202 via internal connections 1220A, 1220B, 1220C and 1220D. Internal connections may implemented as a bus, multiplexor, crossbar switch, and/or any other suitable connection technology.

Inputs and outputs for the processor 1200 may be provided via a bus 1216, which may include one or more conductive lines. The bus 1216 may be communicatively coupled to one or more components of processor 1200, for example the controller 1214, cache 1210, and/or register 1212. The bus 1216 may be coupled to one or more components of the system.

The bus 1216 may be coupled to one or more external memories. The external memories may include Read Only Memory (ROM) 1232. ROM 1232 may be a masked ROM, Electronically Programmable Read Only Memory (EPROM) or any other suitable technology. The external memory may include Random Access Memory (RAM) 1233. RAM 1233 may be a static RAM, battery backed up static RAM, Dynamic RAM (DRAM) or any other suitable technology. The external memory may include Electrically Erasable Programmable Read Only Memory (EEPROM) 1235. The external memory may include Flash memory 1234. The external memory may include a magnetic storage device such as disc 1236.

In various embodiments where components, systems and/or methods are implemented using a programmable device, such as a computer-based system or programmable logic, it should be appreciated that the above-described systems and methods can be implemented using any of various known or later developed programming languages, such as "C", "C++", "FORTRAN", "Pascal", "VHDL" and the like. Accordingly, various storage media, such as magnetic computer disks, optical disks, electronic memories and the like, can be prepared that can contain information that can direct a device, such as a computer, to implement the above-described systems and/or methods. Once an appropriate device has access to the information and programs contained on the storage media, the storage media can provide the information and programs to the device, thus enabling the device to perform functions of the systems and/or methods described herein. For example, if a computer disk containing appropriate materials, such as a source file, an object file, an executable file or the like, were provided to a computer, the computer could receive the information, appropriately configure itself and perform the functions of the various systems and methods outlined in the diagrams and flowcharts above to implement the various functions. That is, the computer could receive various portions of information from the disk relating to different elements of the above-described systems and/or methods, implement the individual systems and/or methods and coordinate the functions of the individual systems and/or methods described above.

In view of this disclosure it is noted that the various methods and devices described herein can be implemented in hardware, software and firmware. Further, the various methods and parameters are included by way of example only and not in any limiting sense. In view of this disclosure, those of ordinary skill in the art can implement the present teachings in determining their own techniques and needed equipment to affect these techniques, while remaining within the scope of the invention. The functionality of one or more of the processors described herein may be incorporated into a fewer number or a single processing unit (e.g., a CPU) and may be implemented using application specific integrated circuits (ASICs) or general purpose processing circuits which are programmed responsive to executable instruction to perform the functions described herein.

Of course, it is to be appreciated that any one of the examples, embodiments or processes described herein may be combined with one or more other examples, embodiments and/or processes or be separated and/or performed amongst separate devices or device portions in accordance with the present systems, devices and methods.

Finally, the above-discussion is intended to be merely illustrative of the present system and should not be construed as limiting the appended claims to any particular embodiment or group of embodiments. Thus, while the present system has been described in particular detail with reference to exemplary embodiments, it should also be appreciated that numerous modifications and alternative embodiments may be devised by those having ordinary skill in the art without departing from the broader and intended spirit and scope of the present system as set forth in the claims that follow. Accordingly, the specification and drawings are to be regarded in an illustrative manner and are not intended to limit the scope of the appended claims.

## Claims

1. A computer-implemented method comprising:
displaying a first clinical report (308) having a type, wherein the first clinical report is in an unstructured electronic file format;
displaying, via a Graphical User Interface, a skeleton report template (302a) that enables a user to select, from a plurality of elements (304a) of a reference information model, a subset of the plurality of elements for inclusion in a custom data model for clinical reports of the type, wherein the Graphical User Interface is further configured to enable the user to map non-machine readable information from the first clinical report (308) to the elements of the custom data model whereby the non-machine readable information is extracted from the first clinical report and stored in machine-readable form in a first converted clinical report compliant with the reference information model; and
parsing a second clinical report of the type using the custom data model to generate a second converted clinical report compliant with the reference information model that contains, in machine-readable form, non-machine-readable information from the second clinical report.

2. The method of claim 1, wherein the parsing of a second clinical report of the type includes selecting the custom data model and the second clinical report, and information from the first converted clinical report, in connection with the custom data model, to automatically extract the non-machine readable information from the second clinical report and store the extracted information in machine-readable form in a second converted clinical report compliant with the reference information model.

3. The method of claim 1 or 2, wherein the parsing of a second clinical report of the type includes displaying the second clinical report, and, upon selection of the custom data model, enabling the Graphical User Interface for mapping, responsive to user inputs, non-machine-readable information from the second clinical report to the elements of the custom data model for generating the second converted clinical report.

4. A computing system comprising at least one processor and at least one memory storing instructions which when executed by the at least one processor cause the computing system to:
display a graphical user interface configured to enable a user to select clinical information fields stored in a default data model;
display a first clinical report document of a given type via a graphical user interface, the first clinical report containing corresponding clinical information fields; and
store computer-readable instructions for implementing a data ingestion tool and a data model generator via the processor;
wherein the data model generator is configured to generate a clinical report data model by guiding a user through a sequential report mapping process,
wherein the data ingestion tool is configured to utilize the clinical report data model to guide the user through a streamlined mapping process upon receipt of additional clinical reports.

5. The system of claim 4, wherein the sequential report mapping process involves prompting the user, via the graphical user interface, to select the clinical information fields stored in the default data model and map the clinical information fields to the corresponding clinical information fields embodied in the first clinical report.

6. The system of claim 4 or 5, wherein the clinical information fields comprise patient information, clinical test results, diagnoses, symptoms, genetic mutations, treatments, and/or patient outcomes.

7. The system of claims 4-6, wherein mapping the clinical information fields to the corresponding clinical information fields comprises determining coordinates of the clinical information fields within the first clinical report.

8. The system of claims 4-7, wherein mapping the clinical information fields to the corresponding clinical information fields comprises determining relative positions between the corresponding clinical information fields within the first clinical report.

9. The system of claims 4-8, wherein the data model generator is further configured to prompt the user to assign an information field as an anchor point from which each of the remaining clinical information fields is mapped.

10. The system of claims 5-9, wherein mapping the clinical information fields to the corresponding clinical information fields comprises determining font attributes of the information fields within the first clinical report.

11. The system of claims 4-10, wherein the clinical report data model comprises a computer-readable model compatible with all clinical reports having the same document structure as the clinical report documents.

12. The system of claims 4-11, wherein the sequential report mapping process involves prompting the user, via the graphical user interface, to indicate whether the clinical information fields are required or optional.

13. The system of claims 4-12, wherein the first clinical report comprises genomics reports and at least one of the corresponding clinical information fields comprises a genetic mutation.

14. A method of modeling and processing clinical report data, the method comprising:
receiving and displaying clinical report documents on a graphical user interface of the computing device;
generating a clinical report data model by guiding a user through a sequential report mapping process, wherein the sequential report matting process includes prompting the user, via the graphical user interface, to select clinical information fields stored in a default data model and map the clinical information fields to corresponding clinical information fields embodied in the clinical report documents; and
utilizing the clinical report data model to guide the user through a streamlined mapping process upon receipt of additional clinical reports.

15. A computer program product comprising instructions which, when the program is executed by a computer, cause the computer to carry out the method of claims 1-4, 14.
